# EUROPEAN PATENT SPECIFICATION

(11) **EP 4 389 396 B1**
(45) Date of publication and mention of the grant of the patent: **19.11.2025**
(21) Application number: 22214431.3
(22) Date of filing: 19.12.2022
(51) Int. Cl.: B29C 64/227, B29C 64/106, B29C 64/245, B33Y 30/00, C12M 1/26

(54) **UNDERWATER 3D BIOPRINTER COMPRISING A MODULAR SYSTEM**
UNTERWASSER-3D-BIODRUCKER MIT EINEM MODULAREN SYSTEM
BIO-IMPRIMANTE 3D SOUS-MARINE COMPRENANT UN SYSTÈME MODULAIRE

(43) Date of publication of application: 26.06.2024
(73) Proprietor: BIOPRINT3RS S.R.L., 40138 Bologna (IT)
(72) Inventor: FORESTI, Ruben, 43125 PARMA (IT)
(74) Representative: Dondi, Silvia

(56) References cited:
- WO-A1-2017/023865
- WO-A1-2020/252624
- WO-A1-2022/047563
- CN-A- 112 936 858

## Description

The present invention relates to an underwater 3D bioprinter comprising a modular system.

The invention has application in manipulation and processes in the biological and bioengineering fields, in particular in the production of medical devices by 3D printing (class III implantable devices).

The category of underwater 3D printers, as described for example in document WO2020252624A1, is known.

There are also known 3D bioprinters which carry out an additive manufacturing process that uses synthetic materials or ones of a natural origin, live cells and active biomolecules to fabricate structures that have the same characteristics as natural tissues or are capable of interacting with them. At present, 3D bioprinters are certified, but guaranteeing a certified process for the product of the printing is complex. This is due to the fact that 3D bioprinters cannot be sterilised, but only sanitised, i.e. they cannot be placed in an autoclave (as is required by FDA standards for sterility in the biological sector), and the fact that there is always at least one mobile axis above the print platform. The friction on the mechanisms causes particles to be incorporated within the printed product, which could therefore be contaminated. WO2017/023865 A1 discloses a 3D bioprinter comprising extrusion printing means.

In this context, the technical task at the basis of the present invention is to propose an underwater 3D bioprinter comprising a modular system which overcomes the aforementioned drawbacks of the prior art.

In particular, one object of the present invention is to propose an underwater 3D bioprinter comprising a modular system which are sterilisable and can be immersed in fluids capable of preventing access by external agents in the production process.

Another object of the present invention is to propose an underwater 3D bioprinter comprising a modular system which is capable of printing implantable medical devices (class III).

The stated technical task and the specified objects are substantially achieved by an underwater 3D bioprinter according to the appended claims.

Additional features and advantages of the present invention will emerge more clearly from the approximate and hence non-limiting description of a preferred but not exclusive embodiment of an underwater 3D bioprinter, as illustrated in the accompanying drawings, in which:

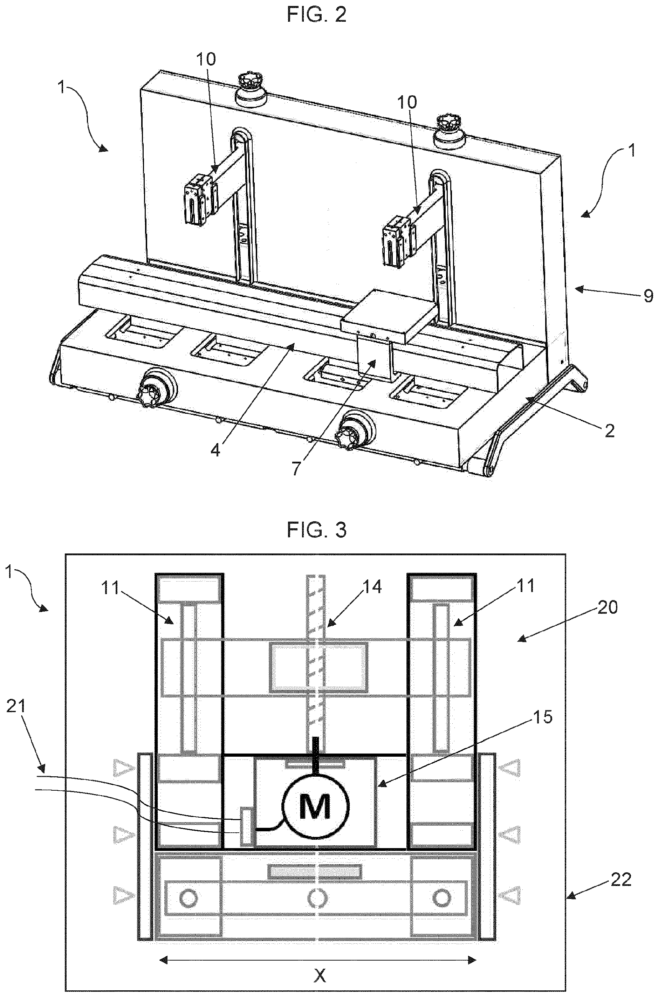

- figures 1 and 2 illustrate two adjacent modular systems usable in an underwater 3D bioprinter of the present invention, respectively in a configuration without and with a coating, in a perspective view;
- figure 3 illustrates a schematic front view of one of the modular systems in figure 1;

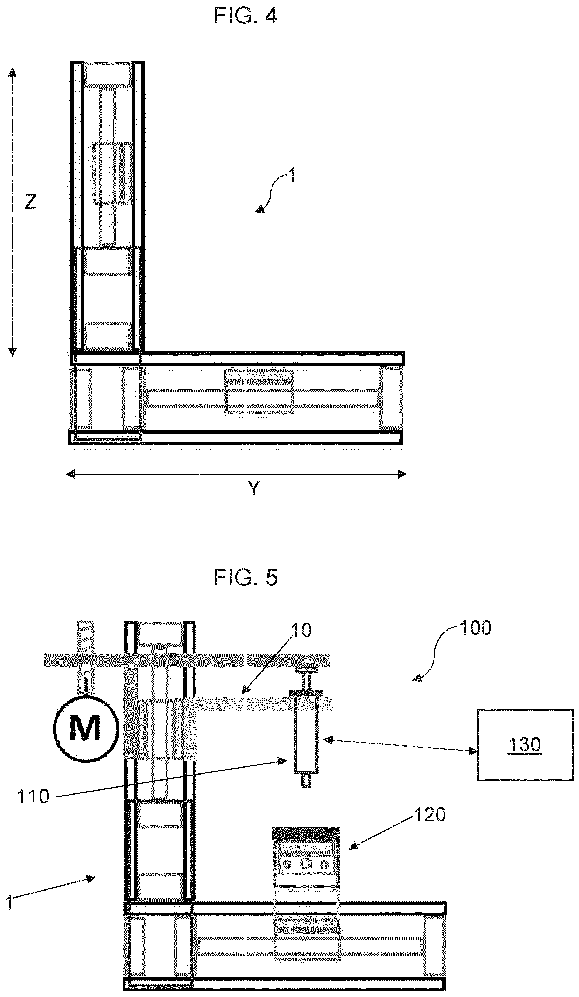

- figure 4 illustrates a schematic side view of one of the modular systems in figure 1;
- figure 5 illustrates a schematic side view of an underwater 3D bioprinter, according to the present invention;
- figure 6 illustrates a schematic view of a (freezing) embodiment of a print platform of the underwater 3D bioprinter in figure 5;
- figures 7a-7e illustrate schematic views of different embodiments of the print platform of the underwater 3D bioprinter in figure 5;

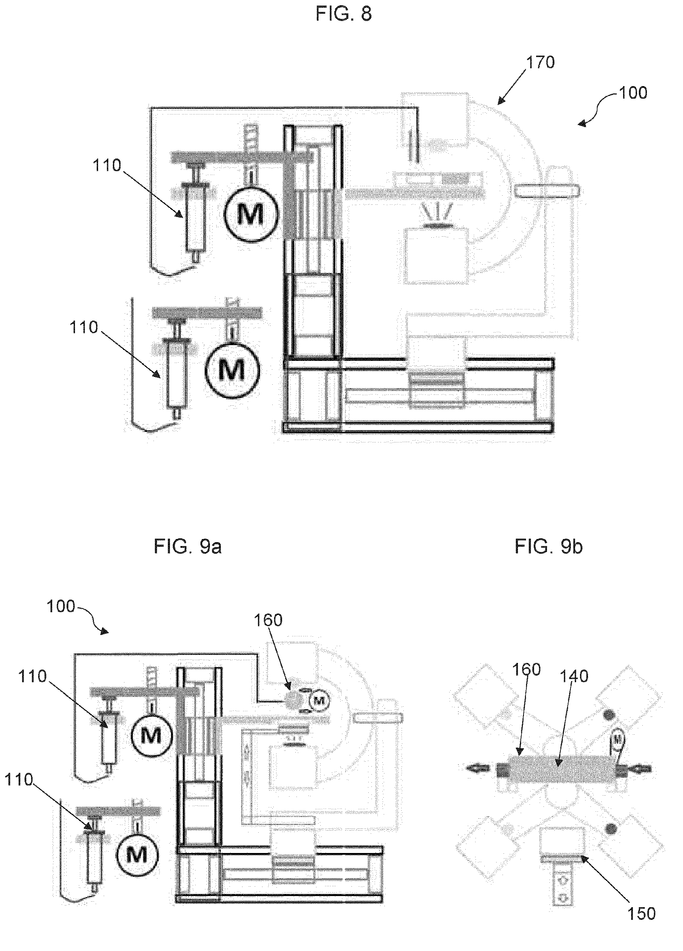

- figure 8 illustrates a schematic side view of an embodiment (with a microscope) of the underwater 3D bioprinter in figure 5;
- figures 9a and 9b respectively illustrate a schematic side view and a schematic top view of an embodiment (change of medium) of the underwater 3D bioprinter in figure 5;
- figures 10a and 10b respectively illustrate a schematic side view and detail of an embodiment (rotary system) of the underwater 3D bioprinter in figure 5;
- figure 11 illustrates a dynamometer, not belonging to the present invention, in a schematic side view;
- figure 12 illustrates a scale, not belonging to the present invention, in a schematic side view.

With reference to the figures, the number 1 denotes a modular system. As will be clearer from the following description, the system in question is a single-axis modular system that can be assembled to produce a final multi-axis device equipped with accessories for conversion into: 3D printer, scale or dynamometer, system for changing the medium intended for cells, microscope equipped with LEDs and/or mixture mixer.

The modular system 1 comprises a base unit 2 in turn comprising a work surface 3. The work surface 3 is movable along a working plane defined by a first direction X and a second direction Y which are inclined relative to each other. The first direction X and the second direction Y are preferably orthogonal to each other. Making reference to a Cartesian system, the first direction is identifiable as the x-axis, and the second direction as the y-axis, or vice versa. The plane in question is thus the xy plane.

Preferably, the modular system 1 comprises a first guide means 4 for guiding the work surface 3 along the first direction X and a second guide means 5 for guiding the work surface 3 along the second direction Y.

Preferably, the base unit 2 comprises a plate 6. The work surface 3 is a surface of the mobile block 6, in particular the upper one.

Preferably, the plate 6 is a solid body, for example a parallelepiped. Preferably, the plate 6 is made of PEEK or steel (for example AISI 316L) or medical grade aluminium.

In the embodiment described and illustrated here, the plate 6 is mounted on the first guide means 4 and the first guide means 4 is in turn mounted on the second guide means 5.

In particular, the first guide means 4 comprises a first guide on which the plate 6 is slidably mounted. In particular, the first guide means 4 comprises a sliding block 7 on which the plate 6 is mounted. The sliding block 7 is slidably mounted on the first guide 4. The sliding block 7 and the first guide 4 define a linear recirculating ball guide.

The second guide means 5 comprises a slide 8 that is slidable in a groove. In accordance with another unillustrated embodiment, the first guides of the first guide means 4 are two parallel cylindrical rods.

The second guides of the second guide means 5 are two cylindrical rods parallel to each other, positioned below the cylindrical rods of the first guide means 4. In particular, the second cylindrical rods are positioned below opposite ends of the first cylindrical rods.

The modular system 1 comprises a support member on which the first guides 4 are positioned and a mobile block bearing the work surface 3. The support member also acts as a support for the mobile block.

The support member is slidably mounted on the second guides. In particular, two opposite ends of the support member are mounted on the second guides.

The work surface 3 can thus move forwards or backwards, to the right or to the left, relative to a generic point of the working plane.

The modular system 1 comprises an upright structure 9 fixed to one side of the base unit 2. The upright structure 9 extends away from the base unit 2.

The modular system 1 comprises a support head 10 for a tool or an accessory. Depending on the tool or accessory that is mounted on the head 10, the modular system 1 can perform one of the previously listed functions. The head 10 thus presents itself as a universal coupling system whereby various functional applications can be obtained.

The head 10 is mounted on the upright structure 9.

The upright structure 9 comprises a guide means 11 for guiding the head 10 along a third direction transversal to the working plane. Preferably, the third direction is orthogonal to the working plane and, consequently, to the first and second directions. In particular, again with reference to a Cartesian system, the third direction is identifiable as the z-axis.

The guide means 11 preferably comprises two columns spaced apart from each other. The upright structure 9 comprises a support 12 on which the head 10 is mounted and constrained to slide along the two columns 11. The support 12 preferably comprises two sleeves 13 at two opposite ends. Each sleeve 13 is constrained to slide along the respective column 11.

The upright structure 9 comprises a sliding means 14 for sliding the support head 10 along the guide means 11.

Preferably, the sliding means 14 is of the screw-nut type, as will be better explained below.

Alternatively, the sliding means 14 can be of the belt-rack or electromagnetic linear actuator type.

The upright structure 9 comprises at least one motor M for the sliding means 14 and a box-like casing 15 provided with sealing gaskets for housing the motor M. This assures the possibility of using the modular system 1 underwater.

The modular system 1 can be inserted into a confined environment 20. The base unit 2 and the upright structure 9 are housed inside the environment 20 together with the components thereof.

The modular system 1 comprises at least one tubular body 21 extending partly inside and partly outside the environment 20. The tubular body 21 defines a tubular cavity adapted for the passage of cables of the motor M. This makes it possible to keep outside the environment 20 all the components that are not strictly essential for the mechanical operation of the final device obtainable by integrating one or more tools or accessories with the modular system 1, such as, for example, a control unit.

For example, the confined environment 20 is a tank filled with at least one fluid. The fluid can be liquid or gaseous. Preferably, the fluid is gaseous. For example, the fluid is VHP or H₂O₂. If the modular system 1 is used for the production of electronic components, in particular microprocessors with subsequently doped biological material, the fluid can be ammonia based.

The final device is of the underwater type, thus allowing the possibility of never removing it from the production environment during the typical highpressure/temperature washing cycles with aggressive chemical agents, or of being able to close and carry it like a briefcase and sanitise it at the destination site prior to use.

For example, if the modular system 1 were immersed in a liquid fluid, the contaminating particles would be stopped on the surface of that liquid; if it were immersed in an ammoniated environment, it could avoid oxidisation of the product, for example sensors, generated by air, or rather by the oxygen contained in it; or else, if it were immersed in vapours that react with the production material, it would be possible to directly interact with the production process.

Alternatively, the modular system 1 can be inserted into an isolation device 22 adapted to delimit a contamination-controlled environment 20. For example, the isolation device 22 is an isolator or a laminar flow hood.

For use in this type of confined environment 20, a coating of the modular system 1 having a roughness of less than 0.5 µm and inclined (>1%) surfaces is conveniently used. In this manner, besides being sterilisable, the modular system 1 can be simply sanitised, for all applications that do not require sterility.

More preferably, the roughness is less than 0.1 µm.

Conveniently, the guide means 11 and the sliding means 14 are made of PTFE or nylon or EPDM or stainless steel or a medical grade inert material. In other words, the material must be self-lubricating and sterilisable, i.e. suitable for being placed in an autoclave.

For sanitisation purposes, the guide means 11 and the sliding means 14 comprise bearings provided with sealing gaskets, i.e. bearings suitable for sanitisation. Alternatively, no bearings are present.

As mentioned above, the sliding means 14 are preferably of the screw-nut type.

In the embodiment described and illustrated here, the sliding means 14 comprises a nut coupled with a corresponding threaded portion of the support 12.

The nut 14 is preferably interposed between the two columns 11.

In the preferred embodiment illustrated in the figures, the upright structure 9 comprises two frame sections 16, each of which houses therewithin one of the two columns 11. The support head 10 is interposed between the two frame sections 16.

Preferably, the upright structure 9, in a front view, is a frame having a square "U" shape; thus, the box-like casing 15 defines the base of the U and the two frame sections 16 constitute the parallel arms of the "U".

The nut 14 is preferably at the centre of the "U"-shaped frame.

The upright structure 9 is rotatably mounted relative to the base unit 2 so that it can be closed over it in a briefcase-like manner. In other words, the upright structure 9 is mounted on the base unit 2 by means of hinges.

The modular system 1 preferably comprises an interconnection portion 17 between the base unit 2 and the upright structure 9. The upright structure 9 is hinged to the interconnection portion 17. For example, the interconnection portion 17 is a flange.

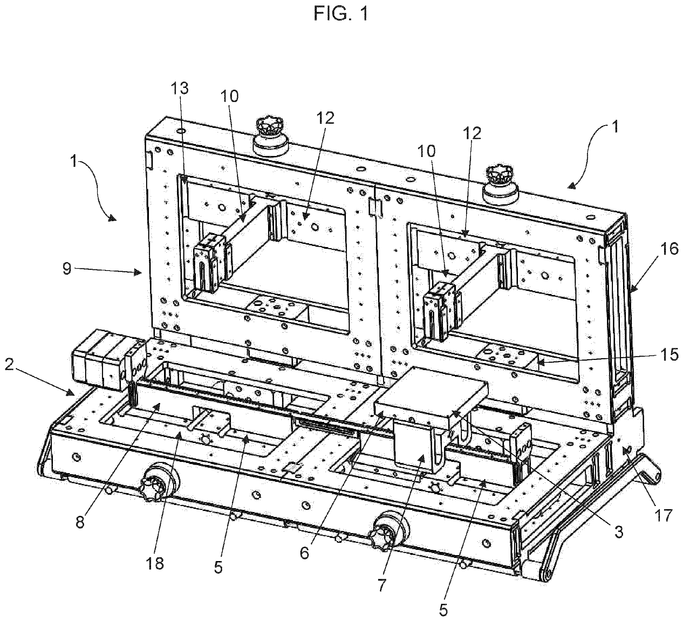

The system 1, as mentioned, is modular. In this regard, figure 1 illustrates two adjacent modular systems 1. They share the work surface 3. In order to obtain this, the first guide means 4 needs to be shared. In this case, the first guide 4 travels over both base units 2 for the entire length (x-axis). The work surface 3 can be rotated on the x-axis or take on any combination useful for modularity.

An underwater 3D bioprinter, subject matter of the present invention, is denoted by the number 100 and is described below.

The underwater 3D bioprinter 100 comprises the modular system 1 and an extrusion printing means 110 mounted on the modular system 1. The extrusion printing means 110 can thus also be introduced into the confined environment 20. The extrusion printing means 110 is known and will not be further described. For example, the extrusion printing means 110 comprises extruders. If the extruders possess motors housed inside the environment 20, each motor is installed in a box-like casing provided with sealing gaskets and is connected to the outside (relative to the environment 20) by means of a tubular cable pass-through body 21.

By way of non-limiting example, the extrusion printing means 110 can comprise: hot/cold syringes, hot/cold filament, ink jet, conduction/insulation platform, suction device/blower, blower and plasma, laser, cutter or blade, light or LED (polymerisation/sanitisation), camera, leveller.

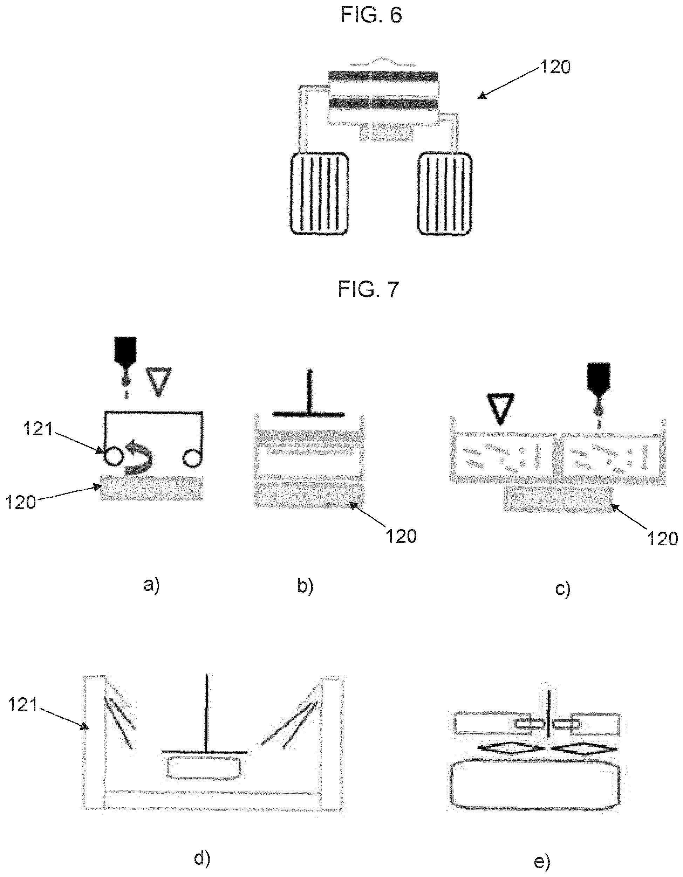

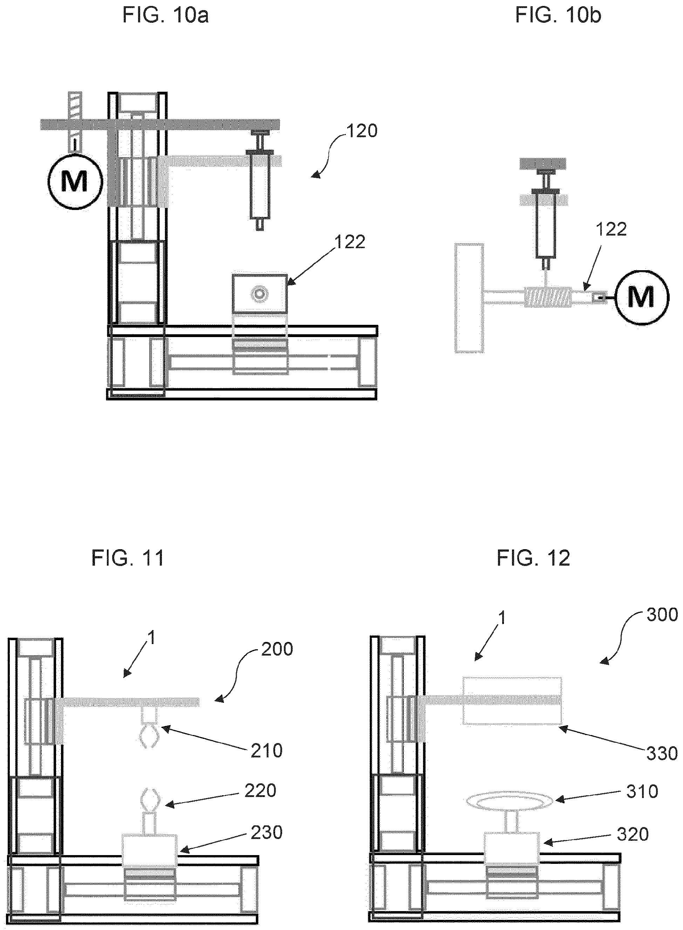

A print platform 120 can be integrated above the work surface 3. Preferably, the print platform 120 can be flat or have a hemispherical shape, as illustrated in figure 6. Alternatively, the print platform 120 can be connected to a rotary system 122 on which to rest the material, as illustrated in figure 10b. Thanks to this embodiment, it is possible to form arteries.

The print platform 120 is preferably of the freezing type and is configured to reach as low as -34 °C in 20 seconds. This preferably takes place in four stages: Peltier, liquid cooling, Peltier, liquid cooling.

Alternatively, the print platform 120 is of the heating type.

Alternatively, the print platform 120 can be provided with a removable customised (shaped, grid) surface.

Alternatively, the print platform 120 can be provided with a sliding roller.

Alternatively, the print platform 120 can comprise a tank with a screen and projector.

Alternatively, the print platform 120 can comprise a tank with powders.

Conveniently, the underwater 3D bioprinter 100 comprises a control unit 130 for controlling the extrusion printing means 110.

The overall structure (sanitisable printer, environmental management system), capable of filtering air, further enables the use of special materials (e.g. multi-component filament which could generate vapours that are toxic for humans).

In accordance with one embodiment, the print platform 120 houses one or more materials intended for the development of a printed product. In such a case, the print platform 120 comprises members or tools 121 for moving or adapting said one or more materials.

The members 121 are for example rollers, flattening systems, layering systems, extruder washing or calibration systems.

Figure 7a illustrates two rollers with a blade and laser/inkjet above. Figure 7b illustrates a tank with a screen and projector. Figure 7c illustrates a tank with powders, with a blade and laser/inkjet above. Figure 7d illustrates a washing and drying system (the washing liquid enters from the left side and air enters from the right side). Figure 7e illustrates a needle levelling and positioning sensor. The needle is vertical and, in a top-down order, we find a contactless sensor, cutting blades and a receptacle for scraps.

In accordance with one embodiment, the underwater 3D bioprinter 100 comprises a culture medium 140. The culture medium 140 can contain cells.

The extruders 110 can be used to carry out a change of medium. If there is a single extruder 110, there can be a selective use to withdraw and introduce medium from and into the culture medium 140.

If there are at least two extruders 110, it will be possible to carry out a continuous recirculation of the culture medium 140. This slow but continuous process prevents the culture medium 140 from being dirtied.

Conveniently, the underwater 3D bioprinter 100 comprises a stimulation system 150 operatively active on the culture medium 140. The stimulation system 150 illustrated is of the optical type, Alternatively, however, it can be thermal, electrical, or electromagnetic, etc.

In the preferred embodiment, the underwater 3D bioprinter 100 comprises a bioreactor 160 in the form of a rotatable tube containing structures dedicated to cell proliferation, including the culture medium 140.

In one embodiment, the underwater 3D bioprinter 100 is equipped with a microscope 170 for observing the cells of the culture medium 140.

A dynamometer, which is not part of the present invention, is denoted by the number 200 and is described below.

The dynamometer 200 comprises the modular system 1 and at least a first gripper 210 mounted on the support head 10 of the modular system 1 and a second gripper 220 mounted on the work surface 3. The dynamometer 200 comprises a detection system 230 interposed between the work surface 3 and the second gripper 220.

A scale, which is not part of the present invention, is denoted by the number 300 and is described below.

The scale 300 comprises the modular system 1 according to what has been described. The scale 300 comprises at least one plate 310 placed above a detection system 320, which is in turn mounted on the work surface 3 of the modular system 1. The scale comprises a cover 330 for protecting from interaction with laminar flow, mounted on the support head 10 of the modular system 1.

In particular, the proposed configuration of a modular system ensures that there are no mobile axes above the print platform: the X- and Y-axes are below the print area, whereas the Z-axis is lateral. The modular system thus presents itself as a single-axis system moved by a motor placed in a casing provided with specific sealing gaskets for sanitisation and located inside a development environment that comprises what is strictly indispensable for the mechanical functioning of the system, while the rest of the components are maintained outside the environment, thanks to the tubular cable pass-through bodies. This assures the possibility of sterilisation in an autoclave.

Furthermore, the fact that the components of the upright structure (Z-axis) may be made of self-lubricating materials makes it possible to prevent friction from producing particles that are incorporated into the printed product and the fact that they are adapted to be placed in an autoclave enables the complete sterilisability thereof.

Moreover, the proposed modular system is versatile, as it can be easily transformed into a plurality of devices with contained additions sharing the same structure as the modular system itself.

Consequently, the 3D bioprinter obtained from the modular system is also for underwater use, as well as being sterilisable.

## Claims

1. An underwater 3D bioprinter (100), comprising:
a modular system (1) in turn comprising:
- a base unit (2) comprising a work surface (3) that is movable along a working plane defined by a first direction (X) and a second direction (Y) which are inclined relative to each other;
- a support head (10) for supporting a tool or an accessory;
- an upright structure (9) constrained to one side of the base unit (2) and extending away therefrom, said upright structure (9) being rotatably mounted relative to the base unit (2) by means of hinges so as to be able to be closed on top of it in a briefcase-like manner, said upright structure (9) comprising a guide means (11) for guiding the support head (10) along a third direction (Z) transversal to the working plane, a sliding means (14) for sliding the support head (10) along the guide means (11), at least one motor (M) operatively active on the sliding means (14) and a box-like casing (15) provided with sealing gaskets for housing said motor (M) so as to assure the possibility of using the modular system (1) underwater;
said modular system (1) further comprising at least one tubular body (21) defining a tubular cavity for cables connected to said motor (M),
said underwater 3D bioprinter (100) further comprising an extrusion printing means (110) mounted on the support head (10) of the modular system (1);
a print platform (120) mounted on the work surface (3);
a control unit (130) of the extrusion printing means (110).

2. The underwater 3D bioprinter (100) according to claim 1, wherein the print platform (120) is flat or has a hemispherical shape.

3. The underwater 3D bioprinter (100) according to claim 1, comprising a culture medium (140), a stimulation system (150) for stimulating the culture medium (140), said extrusion printing means (110) comprising one or more extruders configured to introduce medium into the culture medium (140).

4. The underwater 3D bioprinter (100) according to any one of claims 1 to 3, wherein said print platform (120) houses one or more materials intended for the development of a printed product, said print platform (120) further comprising members or tools (121) for moving or adapting said one or more materials, said members (121) being chosen among: rollers, flattening systems, layering systems, extruder washing or calibration systems.

5. The underwater 3D bioprinter (100) according to any one of the preceding claims, wherein the guide means (11) and the sliding means (14) are made of PTFE or nylon or EPDM or stainless steel inox or a medical grade inert material.

6. The underwater 3D bioprinter (100) according to any one of the preceding claims, wherein the guide means (11) comprises two columns spaced apart from each other and a support (12) on which the head (10) is mounted, said support (12) being interposed between the columns (11) and constrained to slide along them, said sliding means (14) comprising a nut coupled to a corresponding threaded portion of the support (12).

7. The underwater 3D bioprinter (100) according to any one of the preceding claims, wherein said base unit (2) comprises:
- a plate (6);
- a first guide means (4) for guiding the plate (6) along the first direction (X);
- a second guide means (5) for guiding the plate (6) along the second direction (Y), said work surface (3) being a surface of said plate (6).

8. The underwater 3D bioprinter (100) according to claim 7, wherein said plate (6) is mounted on the first guide means (4) and the first guide means (4) is in turn mounted on the second guide means (5).

9. The underwater 3D bioprinter (100) according to any one of the preceding claims, wherein the base unit (2) and the upright structure (9) comprise a coating having a roughness of less than 0.5 µm.

## Patentansprüche

1. Unterwasser-3D-Biodrucker (100), umfassend:
ein modulares System (1), das wiederum Folgendes umfasst:
- eine Basiseinheit (2), die eine Arbeitsoberfläche (3) umfasst, die entlang einer Arbeitsebene beweglich ist, die durch eine erste Richtung (X) und eine zweite Richtung (Y) definiert ist, die relativ zueinander geneigt sind;
- einen Trägerkopf (10) zum Tragen eines Werkzeugs oder eines Zubehörteils;
- eine aufrecht stehende Struktur (9), die an einer Seite der Basiseinheit (2) gefesselt ist und sich von dieser wegführend erstreckt, wobei die aufrecht stehende Struktur (9) relativ zur Basiseinheit (2) mittels Scharnieren drehbar montiert ist, so dass sie oben darauf in einer aktenkofferartigen Weise geschlossen werden kann, wobei die aufrecht stehende Struktur (9) Führungsmittel (11) zum Führen des Trägerkopfes (10) entlang einer dritten Richtung (Z) quer zur Arbeitsebene, Gleitmittel (14) zum Gleiten des Trägerkopfes (10) entlang der Führungsmittel (11), mindestens einen Motor (M), der auf den Gleitmitteln (14) betriebswirksam ist, und ein kastenartiges Gehäuse (15) umfasst, das mit Versiegelungsdichtungen zur Aufnahme des Motors (M) versehen ist, um die Möglichkeit der Verwendung des modularen Systems (1) unter Wasser sicherzustellen;
wobei das modulare System (1) ferner mindestens einen rohrförmigen Körper (21) umfasst, der einen rohrförmigen Hohlraum für Kabel definiert, die mit dem Motor (M) verbunden sind,
wobei der Unterwasser-3D-Biodrucker (100) ferner Extrusionsdruckmittel (110) umfasst, die an dem Trägerkopf (10) des modularen Systems (1) montiert sind;
eine Druckplattform (120), die auf der Arbeitsoberfläche (3) montiert ist;
eine Steuereinheit (130) der Extrusionsdruckmittel (110).

2. Unterwasser-3D-Biodrucker (100) nach Anspruch 1, wobei die Druckplattform (120) flach ist oder eine halbkugelförmige Form aufweist.

3. Unterwasser-3D-Biodrucker (100) nach Anspruch 1, umfassend ein Kulturmedium (140), ein Stimulationssystem (150) zum Stimulieren des Kulturmediums (140), wobei die Extrusionsdruckmittel (110) einen oder mehrere Extruder umfassen, die ausgelegt sind, um Medium in das Kulturmedium (140) einzubringen.

4. Unterwasser-3D-Biodrucker (100) nach einem der Ansprüche 1 bis 3, wobei die Druckplattform (120) ein oder mehrere Materialien aufnimmt, die für die Entwicklung eines Druckprodukts bestimmt sind, wobei die Druckplattform (120) ferner Elemente oder Werkzeuge (121) zum Bewegen oder Anpassen des einen oder der mehreren Materialien umfasst, wobei die Elemente (121) ausgewählt sind aus: Walzen, Abflachungssystemen, Schichtsystemen, Extruderwasch- oder Kalibriersystemen.

5. Unterwasser-3D-Biodrucker (100) nach einem der vorhergehenden Ansprüche, wobei die Führungsmittel (11) und die Gleitmittel (14) aus PTFE oder Nylon oder EPDM oder nichtrostendem Stahl oder einem inerten Material medizinischer Qualität hergestellt sind.

6. Unterwasser-3D-Biodrucker (100) nach einem der vorhergehenden Ansprüche, wobei die Führungsmittel (11) zwei voneinander beabstandete Säulen und einen Träger (12), auf dem der Kopf (10) montiert ist, umfassen, wobei der Träger (12) zwischen den Säulen (11) angeordnet und gezwungen ist, entlang dieser zu gleiten, wobei die Gleitmittel (14) eine Mutter umfassen, die mit einem entsprechenden Gewindeabschnitt des Trägers (12) gekoppelt ist.

7. Unterwasser-3D-Biodrucker (100) nach einem der vorhergehenden Ansprüche, wobei die Basiseinheit (2) Folgendes umfasst:
- eine Platte (6);
- erste Führungsmittel (4) zum Führen der Platte (6) entlang der ersten Richtung (X);
- zweite Führungsmittel (5) zum Führen der Platte (6) entlang der zweiten Richtung (Y), wobei die Arbeitsoberfläche (3) eine Oberfläche der Platte (6) ist.

8. Unterwasser-3D-Biodrucker (100) nach Anspruch 7, wobei die Platte (6) an den ersten Führungsmitteln (4) montiert ist und die ersten Führungsmittel (4) wiederum an den zweiten Führungsmitteln (5) montiert sind.

9. Unterwasser-3D-Biodrucker (100) nach einem der vorhergehenden Ansprüche, wobei die Basiseinheit (2) und die aufrecht stehende Struktur (9) eine Beschichtung mit einer Rauheit von weniger als 0,5 µm umfassen.

## Revendications

1. Bio-imprimante 3D sous-marine (100), comprenant :
un système modulaire (1), comprenant à son tour :
- une unité de base (2), comprenant une surface de travail (3) qui est mobile le long d'un plan de travail défini par une première direction (X) et une deuxième direction (Y) qui sont inclinées l'une par rapport à l'autre ;
- une tête de support (10) pour supporter un outil ou un accessoire ;
- une structure verticale (9) contrainte d'un côté de l'unité de base (2) et s'étendant en s'éloignant de celle-ci, ladite structure verticale (9) étant montée de manière rotative par rapport à l'unité de base (2) au moyen de charnières de sorte à pouvoir se refermer au sommet de celle-ci à la manière d'une mallette, ladite structure verticale (9) comprenant des moyens de guidage (11) pour guider la tête de support (10) le long d'une troisième direction (Z) transversale au plan de travail, des moyens coulissants (14) pour faire coulisser la tête de support (10) le long des moyens de guidage (11), au moins un moteur (M) fonctionnellement actif sur les moyens coulissants (14) et un boîtier (15) en forme de boîte pourvu de joints d'étanchéité pour loger ledit moteur (M) de sorte à garantir la possibilité d'utiliser le système modulaire (1) sous l'eau ;
ledit système modulaire (1) comprenant en outre au moins un corps tubulaire (21) définissant une cavité tubulaire pour des câbles reliés audit moteur (M),
ladite bio-imprimante 3D sous-marine (100) comprenant en outre des moyens d'impression par extrusion (110) montés sur la tête de support (10) du système modulaire (1) ;
une plateforme d'impression (120) montée sur la surface de travail (3) ;
une unité de commande (130) des moyens d'impression par extrusion (110).

2. Bio-imprimante 3D sous-marine (100) selon la revendication 1, dans laquelle la plateforme d'impression (120) est plate ou a une forme hémisphérique.

3. Bio-imprimante 3D sous-marine (100) selon la revendication 1, comprenant un milieu de culture (140), un système de stimulation (150) pour stimuler le milieu de culture (140), lesdits moyens d'impression par extrusion (110) comprenant une ou plusieurs extrudeuses configurées pour introduire un milieu dans le milieu de culture (140).

4. Bio-imprimante 3D sous-marine (100) selon l'une quelconque des revendications 1 à 3, dans laquelle ladite plateforme d'impression (120) loge un ou plusieurs matériaux destinés au développement d'un produit imprimé, ladite plateforme d'impression (120) comprenant en outre des éléments ou des outils (121) pour déplacer ou adapter ledit ou lesdits matériaux, lesdits éléments (121) étant choisis parmi : des rouleaux, des systèmes d'aplatissement, des systèmes de stratification, des systèmes de lavage ou d'étalonnage d'extrudeuse.

5. Bio-imprimante 3D sous-marine (100) selon l'une quelconque des revendications précédentes, dans laquelle les moyens de guidage (11) et les moyens coulissants (14) sont réalisés en PTFE ou en nylon ou en EPDM ou en acier inoxydable ou en un matériau inerte de qualité médicale.

6. Bio-imprimante 3D sous-marine (100) selon l'une quelconque des revendications précédentes, dans laquelle les moyens de guidage (11) comprennent deux colonnes espacées l'une de l'autre et un support (12) sur lequel est montée la tête (10), ledit support (12) étant interposé entre les colonnes (11) et contraint de coulisser le long de celles-ci, lesdits moyens coulissants (14) comprenant un écrou couplé à une partie filetée correspondante du support (12).

7. Bio-imprimante 3D sous-marine (100) selon l'une quelconque des revendications précédentes, dans laquelle ladite unité de base (2) comprend :
- une plaque (6) ;
- des premiers moyens de guidage (4) pour guider la plaque (6) le long de la première direction (X) ;
- des seconds moyens de guidage (5) pour guider la plaque (6) le long de la deuxième direction (Y), ladite surface de travail (3) étant une surface de ladite plaque (6).

8. Bio-imprimante 3D sous-marine (100) selon la revendication 7, dans laquelle ladite plaque (6) est montée sur les premiers moyens de guidage (4) et les premiers moyens de guidage (4) sont à leur tour montés sur les seconds moyens de guidage (5).

9. Bio-imprimante 3D sous-marine (100) selon l'une quelconque des revendications précédentes, dans laquelle l'unité de base (2) et la structure verticale (9) comprennent un revêtement ayant une rugosité inférieure à 0,5 µm.
